(19) Europäisches Patentamt
European Patent Office
Office européen des brevets

(11) Veröffentlichungsnummer: **0 003 297**
**B1**

(12) **EUROPÄISCHE PATENTSCHRIFT**

(45) Veröffentlichungstag der Patentschrift: **13.05.81**

(21) Anmeldenummer: **79100092.0**

(22) Anmeldetag: **12.01.79**

(51) Int. Cl.³: **C 07 D 263/10, C 07 D 263/14, A 01 N 43/76**

(54) **Neue Phenoxy-alkyl-oxazoline, deren Herstellung, sie enthaltende herbizide Mittel und deren Verwendung.**

(30) Priorität: **25.01.78 CH 804/78**

(43) Veröffentlichungstag der Anmeldung:
**08.08.79 Patentblatt 79/16**

(45) Bekanntmachung des Hinweises auf die Patenterteilung:
**13.05.81 Patentblatt 81/19**

(84) Benannte Vertragsstaaten:
**BE CH DE FR GB IT NL**

(56) Entgegenhaltungen:
**US - A - 3 778 445**

(73) Patentinhaber: **CIBA-GEIGY AG**
**Patentabteilung Postfach**
**CH-4002 Basel (CH)**

(72) Erfinder: **Föry, Werner, Dr.**
**Benkenstrasse 65**
**CH-4054 Basel (CH)**
Erfinder: **Böhner, Beat, Dr.**
**Hügelweg 3**
**CH-4102 Binningen (CH)**
Erfinder: **Dürr, Dieter, Dr.**
**Brändelistalweg 16**
**CH-4103 Bottmingen (CH)**
Erfinder: **Pissiotas, Georg, Dr.**
**Breslauerstrasse 8**
**D-7850 Lörrach (DE)**
Erfinder: **Rohr, Otto, Dr.**
**Kilbertweg 19**
**CH-4106 Therwil (CH)**
Erfinder: **Szczepanski, Henry, Dr.**
**Waldshuterstrasse 55**
**CH-4310 Rheinfelden (CH)**

**EP 0 003 297 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Phenoxy-alkyl-oxazoline, deren Herstellung, sie enthaltende herbizide Mittel und deren Verwendung

Die vorliegende Erfindung betrifft neue, herbizid und pflanzenregulatorisch wirksame kernsubstituierte Phenoxy-alkyl-oxazoline, Verfahren zu ihrer Herstellung, ferner herbizide Mittel, die diese neuen Verbindungen als Wirkstoffe enthalten, sowie deren Verwendung zur selektiven Bekämpfung von Unkräutern in Kulturpflanzungen und zur Regulierung des Pflanzenwachstums.

Die neuen Wirkstoffe entsprechen der Formel I

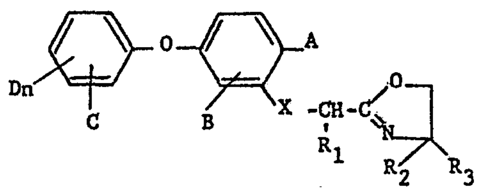

(I)

In dieser Formel bedeuten

A Wasserstoff, ein Halogenatom, die Cyano- oder Nitrogruppe, den Amido- oder Thiamidorest $—CONH_2$ oder $—CSNH_2$,
B Wasserstoff, ein Halogenatom oder eine $C_1—C_4$ Alkylgruppe,
C Halogen, eine Cyano-, Nitro- oder Trifluormethylgruppe, den Amido oder Thiamidorest,
D ein Halogenatom, die Cyano- oder Nitrogruppe,
n eine Zahl 0, 1 oder 2,
$R_1$, $R_2$ und $R_3$ unabhängig voneinander je Wasserstoff oder eine $C_1—C_4$ Alkylgruppe,
X Sauerstoff, Schwefel, eine Sulfinyl- oder Sulfonylgruppe —SO— oder $—SO_2—$.

Aus der US Patentschrift No. 3 877 921 und der DOS 2 613 697 ist bekannt, dass man Phenoxy-alkyloxazoline, resp. Phenoxy-phenoxy-alkyl-tetrazole als Herbizide und Wuchsregulatoren verwenden kann.

Es wurde nun überraschenderweise gefunden, dass die neuen Phenoxy-alkyl-oxazoline der Formel I, sich ausgezeichnet zur Bekämpfung von dikotylen Unkräutern in vorwiegend monokotylen Kulturen wie Getreide, (Weizen, Gerste, Sorghum), Reis, Mais aber auch vereinzelt in dikotylen Kulturen, wie Zuckerrüben oder Soja eignen.

Es wurde eine besonders gute Verträglichkeit gegenüber Reis festgestellt, sowohl gegen "Trockenreis" wie auch gegenüber verpflanztem "Wasserreis."

Die Verbindungen der Formel I sind unter anderem gegen folgende dicotyle Unkräuter wirksam: Sinapis alba, Sida spinosa, Sesbania exaltata, Ipomoea purpurea, Galium aparine Chrysanthemum leucum, Abutilon, Solanum nigrum, Ammania indica, Rotala indica etc.

Die neuen Verbindungen wirken zwar auch gut im Vorauflaufverfahren, das Nachauflaufverfahren hat sich jedoch als besonders wirkungsvoll und zweckmässig erwiesen.

Einige der neuen Wirkstoffe eignen sich auch zur Defoliation und Desikkation z.B. von Baumwoll- und Kartoffelpflanzen, kurz bevor deren Ernte.

Die Herstellung der neuen Verbindungen der Formel I erfolgt nach sich bekannten Reaktionen der chemischen Synthese.

Gemäss einem ersten Verfahren werden Phenoxy-alkyl-äthylenimide der Formel II

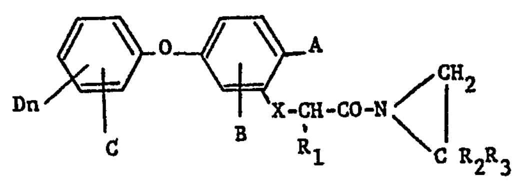

(II)

worin $R_1$, $R_2$, $R_3$, A, B, C, D, n und X die unter Formel I gegebene Bedeutung haben, durch Umlagerung des Aziridinyl Amides unter Einfluss des Jodid-ions in einem Lösungsmittel hergestellt.

Anstelle des Aethylenimides kann auch ein Phenoxy-alkyl-äthylamid der Formel III

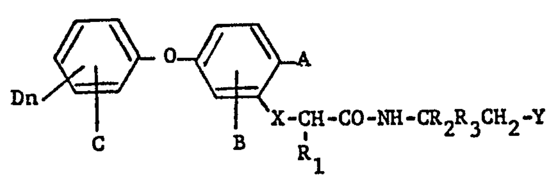

(III)

worin $R_1$, $R_2$, $R_3$, A, B, C, D, n und X die unter Formel I gegebene Bedeutung haben, während Y die OH-Gruppe, ein Halogenatom oder ein Sulfonsäureesterrest sein kann, in einem Lösungsmittel einem Ringschluss unterworfen werden.

Als Lösungsmittel kommen vor allem aprotische wasserlösliche in Frage, wie niedermolekulare Alkohole, Ketone, Dimethylformamid, Dimethylsulfoxyd aber auch chlorierte Kohlenwasserstoffe.

Der Ringschluss erfolgt in Gegenwart einer Base wie z.B. einem Alkalimetallhydroxyd oder einem quaternären Ammoniumhydroxyd, wenn Y ein Halogenatom oder einen Sulfonsäurerest bedeutet, jedoch unter sauren Bedingungen, z.B. in Gegenwart von Schwefelsäure, wenn Y die Hydroxylgruppe bedeutet.

Als Katalysator für den Ringschluss kommen die Alkalimetallsalze oder tertiären Ammoniumsalze von Halogeniden und Sulfonsäuren in Frage.

Der Ringschluss erfolgt bereits bei Raumtemperatur, zur Beschleunigung des Vorganges kann das Reaktions-gemisch bis zum Sieden erwärmt werden.

Ein weiterer Weg, die Phenoxy-alkyl-oxazoline der Formel I herzustellen, besteht darin, dass man ein meta-Phenoxy-phenol der Formel IV

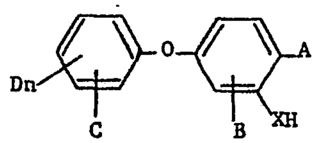

(IV)

worin A, B, C, D und X die unter Formel I gegebene Bedeutung haben, mit einem 2-Oxazolin-alkyl-halogenid der Formel V

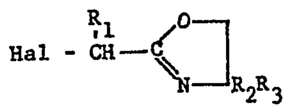

(V)

worin Hal ein Halogenatom bedeutet und $R_1$, $R_2$ und $R_3$ die unter Formel I gegebene Bedeutung haben, in Gegenwart eines säurebindenden Mittels kondensiert

Diese Reaktion wird ebenfalls in einem mit Wasser mischbaren Lösungsmittel oder aber in einem Halogenkohlenwasserstoff vorgenommen, bei Normaldruck und einer Temperatur die zwischen Raumtemperatur und dem Siedepunkt des Reaktionsgemisches liegen kann. Vorzugsweise wird dabei unter Rückfluss gekocht.

Als säurebindendes Mittel können wässerige Alkalimetallhydroxyde wie KOH und NaOH, sowie andere basische Stoffe wie Ammoniak, Karbonate, ($K_2CO_3$, $NaHCO_3$) Alkoholate ($NaOCH_3$, K-tert.butylat) aber auch organische Basen wie Triäthylamin etc. verwendet werden. Falls als Lösungsmittel bereits eine organische Base z.B. Pyridin verwendet wird, so dient dieses zugleich als säurebindendes Mittel.

Die Ausgangsstoffe der Formel II sind am besten durch Amidierung aus den entsprechenden Phenoxy-phenoxy-propionsäuren, wie sie z.B. im schweizerischen Patentgesuch Nr. 2867/77 beschrieben worden sind, erhältlich.

Die Amidierung geschieht nach an sich bekannten Methoden durch Umsetzen der Halogenide oder des Anhydrides dieser Säuren mit Aethylenimin, den entsprechenden Halogenäthylaminen oder einem 2-Amino-äthyl-sulfonat.

Die Ausgangsstoffe der Formel V sind bekannt oder können nach üblichen Verfahren leicht hergestellt werden. Ebenso sind viele Ausgangs-Phenole der Formel IV bereits bekannt.

Noch nicht beschriebene Phenoxyphenole der Formel IV lassen sich nach üblichen Methoden und Techniken leicht herstellen, wie z.B. in DOS 2 433 und 2 433 067 beschrieben ist.

Ausgangsphenole der Formel IV, worin A die Cyanogruppe oder ein Halogenatom darstellt, werden vorteilhaft aus den entsprechenden Nitroverbindungen (Am. Soc. *52*, 1208 (1930) bezw. aus den durch Reducktion daraus erhaltenen aromatischen Aminen über die Diazoniumsalze hergestellt.

So kann man z.B. einen 2-Methoxy-4-chlor-nitro-benzol der Formel

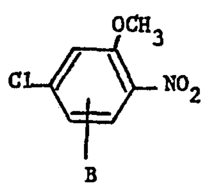

(VI)

worin B die unter Formel I gegebene Bedeutung hat mit einem Phenol der Formel VII

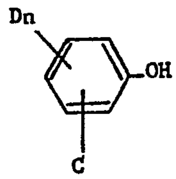

(VII)

worin C, D und n die unter Formel I gegebene Bedeutung haben, in alkalischem Milieu zur Verbindung der Formel VIII

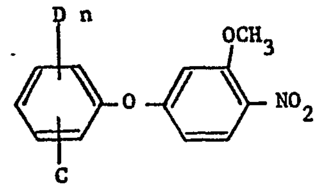

(VIII)

worin C, D und n die unter Formel I gegebene Bedeutung haben, umsetzen, z.B. entsprechend den Angaben in DOS 2 304 006.

Dieselbe Nitroverbindung erhält man auch durch Umsetzung von 2 Mol eines Phenols der Formel VII mit einem 2,4-Dichlornitrobenzol, wobei als Zwischenprodukt eine Verbindung

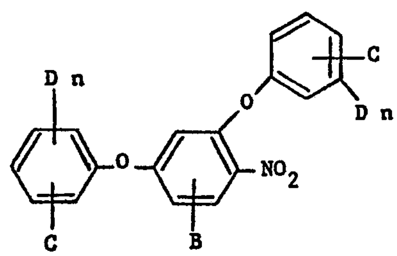

worin B, C, D und n die unter Formel I gegebene Bedeutung haben entsteht, die dann durch Erhitzen mit Methanol und KOH in Dioxan zur Methoxyverbindung VIII "umgeäthert" wird (Methode beschrieben in DOS 2 533 172).

Das so erhaltene nitrierte substituierte Zwischenprodukt wird durch eine übliche Reduktion der Nitrogruppe zum entsprechenden. Amin umgesetzt, das anschliessend in das Diazoniumsalz (z.B. Diazoniumchlorid) überführt wird. Schliesslich wird die Diazogruppe des Diazoniumsalzes nach üblichen Methoden durch die Cyanogruppe ein Halogenatom oder einen anderen Substituenten gemäss der Bedeutung von A ersetzt.

Die Reduktion der Nitrogruppe in Verbindungen der Formel VII wird entweder mit Wasserstoff katalytisch (z.B. mit Raney-Nickel) in Lösung in einem inerten Lösungsmittel oder durch allmäliches Zusetzen von einem 2-Oxazolin-alkyl-halogenid der Formel V zu einem Gemisch aus Eisenpulver und verdünnter Salzsäure bei erhöhter Temperatur durchgeführt.

Die Diazotierung des erhaltenen Amins erfolgt in üblicher Weise in Lösung in verdünnter Salzsäure durch tropfenweise Zugabe einer wässerigen $NaNO_2$-Lösung bei Temperaturen unter 5°C.

Der Austausch der Diazogruppe durch die Cyanogruppe erfolgt durch tropfenweise Zugabe des Diazoniumsalzes zu einer wässerigen Lösung von $K_3$ $[Cu(CN)_4]$, oder durch Zugabe von Kupferpulver und Kupfer-I-cyanid zur Diazoniumsalzlösung. Diese Umsetzungen zu p-Cyanodiphenyläthern ausgehend von substituierten p-Nitro-diphenyläthern sind bereits in DOS 1 912 600 beschrieben worden. Die Cyanogruppe kann anschliessend in die Amido- oder Thiamidogruppe umgewandelt werden.

Der Austausch der Diazogruppe durch Chlor erfolgt durch Eintragen von Kupferchlorür (CuCl) oder fein verteiltem Kupferpulver in die Diazoniumchloridlösung.

Der Ersatz der Diazogruppe durch Brom erfolgt am besten durch Zusatz von KBr und CuBr zu einer Diazoniumsalzlösung, während der Ersatz durch Jod schon durch Einwirkung von Kaliumjodid auf das Diazoniumsalz erfolgen kann.

Um schliesslich daraus das entsprechende freie Ausgangsphenol der Formel IV zu erhalten, wird die metaständige Aetherschutzgruppe ($-O-CH_3$) gespalten, z.B. mittels HBr in Eisessig.

In den nachfolgenden Beispielen wird die Herstellung von einzelnen Phenoxyalkyloxazolinen der Formel I veranschaulicht. Weitere in entsprechender Weise hergestellte Wirkstoffe sind in der anschliessenden Tabelle aufgeführt. Temperaturen sind in Celsius-Graden angegeben.

Beispiel 1

2-[1-[3'-(2''-(2'',4''-Dichlorphenoxy)-6'-chlorphenoxy]-äthyl]-oxazolin

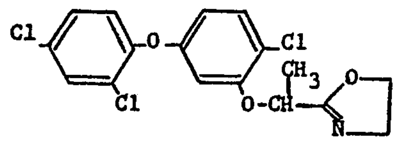

Eine Suspension von 20,3 g 2,4,4' - Trichlor - 3' - hydroxy - diphenyläther, 16,5 g $\alpha$ - Brompropionsäure - 2 - chlor - äthyl - amid und 21,2 g wasserfreiem Kaliumcarbonat in 200 ml Aethylmethylketon wird während 18 Std. bei Rückflusstemperatur gerührt, filtriert und das Filtrat am Vakuum eingedampft. Der ölige Ruckstand (30,1 g) wird in 105 ml Methylenchlorid gelöst, mit 3,57 g Tetrabutylammonium-hydrogensulfat und 35 ml 10%iger, wässeriger Natronlauge versetzt und unter Stickstoffatmosphäre während 2 Std. bei Raumtemperatur gerührt. Die Methylenchloridphase wird abgetrennt, mit Wasser gewaschen, getrocknet und am Vakuum eingedampft. Man erhält 26,4 g der Titelverbindung als Oel, das sich mit wenig Alkohol verrieben zu einer kristallinen Substanz verfestigt. Smp. 85—6°.

Beispiel 2

2-[1-[3'-(2',4''-Dichlorphenoxy)-6'-chlorphenoxy]-äthyl]-4,4-dimethyloxazolin

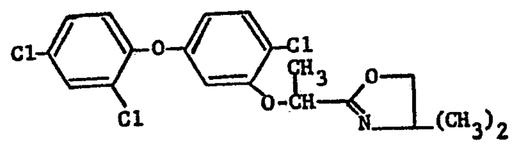

Zu 50 ml Thionylchlorid werden unter Rühren 50 g $\alpha$[ - 3(2',4'-Dichlorphenoxy) - 6 - chlorphenoxy] - propionsäure - 1,1 - dimethyl - 2 - hydroxyäthylamid zugegeben. Nach dem Abklingen der exothermen Reaktion wird 2 Std. bei Raumtemperatur stehengelassen, 100 ml Toluol zugegeben und das Reaktionsgemisch am Vakuum eingedampft. Der Rückstand wird in 250 ml Methylenchlorid gelöst, mit 60 g pulverisiertem Natriumhydroxyd sowie 4 g Benzyltriäthylammoniumchlorid versetzt und 30 Min. unter Stickstoffatmosphäre bei Raumtemperatur gerührt. Die Lösung wird durch Celit filtriert, eingedampft, der Ruckstand in 500 ml Aether gelöst, zweimal mit je 200 ml gesättigter $Na_2CO_3$ — Lösung gewaschen, getrocknet und eingedampft. Man erhält 43.5 g des Oxazolins. Smp. 111—112°.

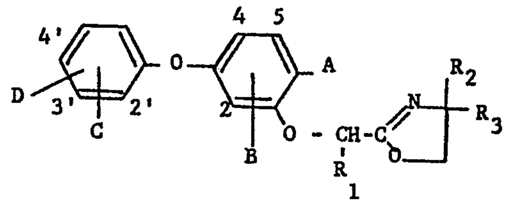

| Verbind. No. | A | B (Pos.) | C (Pos.) | D (Pos.) | $R_1$ | $R_2$ | $R_3$ | Physikal. Konstanten |
|---|---|---|---|---|---|---|---|---|
| 3 | $NO_2$ | | Cl (2′) | Cl (4′) | $CH_3$ | | | Smp. 110–120° |
| 4 | CN | | Cl (2′) | Cl (4′) | $CH_3$ | | | |
| 5 | Br | | Cl (2′) | Cl (4′) | $CH_3$ | | | Smp. 70–80° |
| 6 | Cl | | Cl (2′) | $CF_3$ (4′) | $CH_3$ | | | Smp. 96–98° |
| 7 | Cl | | Cl (2′) | $CF_3$ (4′) | $CH_3$ | $CH_3$ | $CH_3$ | Smp. 55° |
| 8 | $NO_2$ | | Cl (2′) | $CF_3$ (4′) | $CH_3$ | | | $n_D^{35}$ 1.5450 |
| 9 | Cl | | Cl (2′) | Br (4′) | $CH_3$ | | | |
| 10 | Br | | Cl (2′) | Br (4′) | $CH_3$ | | | |
| 11 | CN | | Cl (2′) | $CF_3$ (4′) | $CH_3$ | | | $n_D^{24}$ 1.5340 |
| 12 | Cl | Cl (4) | Cl (2′) | $CF_3$ (4′) | $CH_3$ | | | Oel |
| 13 | | Cl (4) | Cl (2′) | $CF_3$ (4′) | | | | |
| 14 | Cl | Cl (4) | CN (2′) | $CF_3$ (4′) | $CH_3$ | | | Oel |
| 15 | $NO_2$ | Cl (4) | Cl (2′) | $CF_3$ (4′) | $CH_3$ | | | Smp. 50–59° |
| 16 | $NO_2$ | Cl (4) | Cl (2′) | Cl (4′) | $CH_3$ | | | Oel |
| 17 | | $CH_3$ (5) | Cl (2′) | $CF_3$ (4′) | $CH_3$ | | | |
| 18 | Br | | Cl (2′) | $CF_3$ (4′) | $CH_3$ | | | Oel |
| 19 | Cl | | CN (2′) | Cl (4′) | $CH_3$ | | | |
| 20 | CN | | CN (2′) | Cl (4′) | $CH_3$ | | | |
| 21 | $NO_2$ | | CN (2′) | Cl (4′) | $CH_3$ | | | |
| 22 | Cl | | CN (2′) | $CF_3$ (4′) | $CH_3$ | | | Smp. 130–136° |
| 23 | CN | | CN (2′) | $CF_3$ (4′) | $CH_3$ | | | |
| 24 | $NO_2$ | | CN (2′) | $CF_3$ (4′) | $CH_3$ | | | |
| 25 | Cl | | | $CF_3$ (4′) | $CH_3$ | | | Oel |
| 26 | CN | | | $CF_3$ (4′) | $CH_3$ | | | |
| 27 | $NO_2$ | | | $CF_3$ (4′) | $CH_3$ | | | Oel |
| 28 | Cl | | Cl (2′) | Cl (4′) | H | | | |
| 29 | Cl | | Cl (2′) | $CF_3$ (4′) | H | | | |

| Verbind. No. | A | B (Pos.) | C (Pos.) | D (Pos.) | $R_1$ | $R_2$ | $R_3$ | Physikal. Konstanten |
|---|---|---|---|---|---|---|---|---|
| 30 | $NO_2$ | | Cl (2') | $CF_3$ (4') | H | | | |
| 31 | $NO_2$ | | Cl (2') | $Cl_3$ (4') | H | | | |
| 32 | Cl | | Cl (2') | Cl (4') | H | $CH_3$ | $CH_3$ | |
| 33 | $NO_2$ | | CN (2') | | $CH_3$ | $CH_3$ | | Smp. 98–101° |

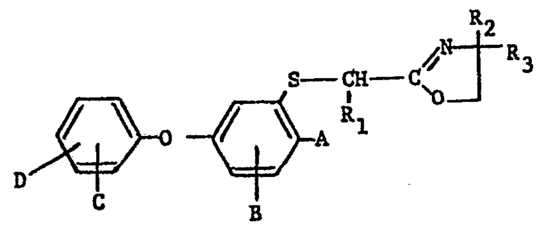

| | | | | | | | | |
|---|---|---|---|---|---|---|---|---|
| 32 | Cl | | Cl (2') | Cl (4') | $CH_3$ | | Oel | |
| 33 | Cl | | Cl (2') | $CF_3$ (4') | $CH_3$ | | Oel | |
| 34 | | | Cl (2') | $CF_3$ (4') | $CH_3$ | | | |
| 35 | | | Cl (2') | Cl (4') | $CH_3$ | | | |

Die neuen Wirkstoffe der Formel I sind stabile Verbindungen, welche in üblichen organischen Lösungsmitteln, wie Alkoholen, Aethern, Ketonen, Dimethylformamid, Dimethylsulfoxyd etc. löslich sind.

Die Herstellung erfindungsgemässer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägniergranulate und Homogengranulate;

In Wasser dispergierbare Wirkstoffkonzentrate:
Spritzpulver (wettable powder), Pasten, Emulsionen; Emulsions-konzentrate

Flüssige Aufarbeitungsformen:
Lösungen

Die Wirkstoffkonzentrationen betragen in den erfindungsgemässen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemässen Mitteln lassen sich andere biozide Wirkstoffe oder Mittel beimischen. Die Herstellung erfindungsgemässer Mittel soll durch die folgenden Formulierungsbeispiele veranschaulicht werden.

Granulat

Zur Herstellung eines 5%igen Granulates werden die folgenden Stoffe verwendet:

5 Teile eines der Wirkstoffe der Formel I,

| | |
|---|---|
| 0,25 | Teile Epichlorhydrin, |
| 0,25 | Teile Cetylpolyglykoläther, |
| 3,50 | Teile Polyäthylenglykol, |
| 91 | Teile Kaolin (Korngrösse: 0,3—08 mm). |

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschliessend im Vacuum verdampft.

Spritzpulver.
Zur Herstellung eines a) 70%igen und b) 10%igen Spritzpulvers werden folgende Bestandteile verwendet:

| | | | |
|---|---|---|---|
| a) | 70 | Teile | eines Wirkstoffes der Formel I |
| | 5 | Teile | Natriumdibutylnaphtylsulfonat |
| | 3 | Teile | Naphthalinsulfonsäuren-Phenolsulfonsäuren-Formaldehyd-Kondensat 3:2:1, |
| | 10 | Teile | Kaolin, |
| | 12 | Teile | Champagne-Kreide; |
| b) | 10 | Teile | eines Wirkstoffes der Formel I |
| | 3 | Teile | Gemisch der Natriumsalze von gesättigten Fettalkoholsulfaten, |
| | 5 | Teile | Naphthalinsulfonsäuren-Formaldehyd-Kondensat, |
| | 82 | Teile | Kaolin. |

Der angegebene Wirkstoff wird auf dei entsprechenden Trägerstoffe (Kaolin und Kreide) aufgezogen und anschliessend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1—80% Wirkstoff erhalten werden, die sich zur Unkrautbekämpfung in Pflanzenkulturen eignen.

Paste
Zur Herstellung einer 45%igen Paste werden folgende Stoffe verwendet:

| | | |
|---|---|---|
| 45 | Teile | eines Wirkstoffes der Formel I, |
| 5 | Teile | Natriumaluminiumsilikat, |
| 14 | Teile | Cetylpolyglykoläther mit 8 Mol Aethylenoxid, |
| 1 | Teil | Oleylpolyglykoläther mit 5 Mol Aethylenoxid, |
| 2 | Teile | Spindeloel, |
| 10 | Teile | Polyäthenglykol, |
| 23 | Teile | Wasser. |

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

Emulsionskonzentrat

Zur Herstellung eines 25%igen Emulsionskonzentrates werden

| | | |
|---|---|---|
| 25 | Teile | eines Wirkstoffes der Formel I, |
| 5 | Teile | einer Mischung von Nonylphenolpolyoxyäthylen oder Calciumdodecylbenzolsulfat, |
| 35 | Teile | 3,3,5-Trimethyl-2-cyclohexen-1-on, |
| 35 | Teile | Dimethylformamid |

miteinander Vermischt. Dieses Konzentrat kann mit Wasser zu Emulsionen auf geeigneten Konzentrationen von z.B. 0,1 bis 10% verdünnt werden. Solche Emulsionen eignen sich zur Bekämpfung von Unkräutern in Kulturpflanzungen.

Die neuen Phenoxyalkyloxyzoline der Formel I sowie die sie enthaltenden Mittel besitzen eine ausgezeichnete selektiv-herbizide Wirkung gegen Unkräuter in verschiedenen, vorzungsweise monokotylen Kulturpflanzenbeständen, ferner üben sie eine das Pflanzenwachstum regulierende Wirkung aus.

Eine besonders bevorzugtes Einsatzgebiet ist die selektive Bekämpfung von vor allem dikotylen Unkräutern z.B. in Getreidekulturen, speziell in Reis, aber auch in Soja.

Obwohl die neuen Wirkstoffe der Formel I bei pre- und post-emergenter Anwendung wirksam sind, scheint die post-emergente Applikation als Kotaktherbizide den Vorzug zu verdienen, obwohl auch der pre-emergente Einsatz von Interesse ist.

Bevorzugt werden die neuen Wirkstoffe als z.B. 25%-ige Spritzpulver oder z.B. 20%-ige emulgierbare Konzentrate formuliert und mit Wasser verdünnt, auf die Pflanzenbestände post-emergent appliziert.

Herbizide Wirkung bei Applikation der Wirkstoffe nach dem Auflaufen der Pflanzen (post-emergent)

Verschiedene Kulturpflanzen und Unkräuter werden aus den Samen in Töpfe im Gewächshaus aufgezogen, bis sie das 4 bis 6-Blatt-Stadium erreicht haben. Dann werden die Pflanzen mit einer wässerigen Wirkstoffemulsion (erhalten aus einem 25%-igen Emulsionskonzentrat) so gespritzt, dass eine Aufwandmenge die 4 kg pro Hektar entspricht verabreicht wurde. Die behandelten Pflanzen werden dann bei optimalen Bedingungen von Licht, Begiessung, Temperatur (24—26°) und Lu ftfeuchtigkeit (45—60% relativ) gehalten. 15 Tage nach Behandlung erfolgte die Auswertung des Versuches. Der Zustand der Pflanzen wurde gemäss folgender Notenskala beurteilt

| | |
|---|---|
| 1 | Pflanze nicht gekeimt oder total abgestorben |
| 2—3 | starke Wirkung |
| 4—6 | mittlere Wirkung |
| 7—8 | geringe Wirkung |
| 9 | keine Wirkung, Pflanze gedeiht wie unbehandelte Kontrolle |
| — | Pflanze mit entsprechendendem Wirkstoff nicht geprüft |

Die Ergebnisse sind in der folgenden Tabelle zusammengefasst.

| Pflanze | geprüfte Verbindung No. 1 | 2 | 6 | 7 | 8 | 22 | 23 | A |
|---|---|---|---|---|---|---|---|---|
| Phaseolus vulgaris | 1 | 3 | 1 | – | 1 | 3 | – | 9 |
| Sinapis alba | 1 | 3 | 1 | 1 | 1 | 1 | 2 | 7 |
| Solanum lycopersicum | 1 | 1 | 1 | 1 | 1 | 1 | 1 | 4 |

Die Verbindung A = 5 - [1' - (para - Trifluoromethylphenoxy-phenoxy) - äthyl]-tetrazol der Formel

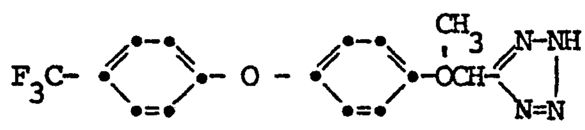

bekannt aus der DOS No. 2 613 697.

In einem weiteren Versuch werden die Pflanzen im 4-bis 6-Blatt-Stadium mit verdünnten Wirkstoffdispersionen in Aufwandmengen von 0,5,1 und 2 kg pro Hektar gespritzt. Die Pflanzen werden dann ebenfalls 15 Tage lang im Gewächshaus bei 24—26°C und 45—60% relativer Luftfeuchtigkeit gehalten bevor der Versuch gemäss der oben gegebenen Notenskala ausgewertet wird. Die Ergebnisse sind in der untenstehenden Tabelle zusammengefasst.

| geprüfte Verbindung | No. 1 | | | 8 | | | 22 | | | B | | |
|---|---|---|---|---|---|---|---|---|---|---|---|---|
| Aufwandmenge kg/ha | 0.5 | 1 | 2 | 0.5 | 1 | 2 | 0.5 | 1 | 2 | 0.5 | 1 | 2 |
| Pflanze | | | | | | | | | | | | |
| Abutilon sp | 2 | 1 | 1 | 1 | 1 | 1 | – | – | 1 | 7 | 6 | 6 |
| Amaranthus retroflexus | 1 | 1 | 1 | 1 | 1 | 1 | – | – | 1 | 3 | 2 | 2 |
| Galium aparine | 3 | 2 | 2 | 2 | 1 | 1 | – | – | 4 | 9 | 8 | 4 |
| Sinapis alba | 2 | 1 | 1 | 1 | 1 | 1 | – | – | 1 | 2 | 2 | 2 |

B = 2-[1'-(4''-Chlor-2''-methyl-phenoxy)-äthyl]-oxazolin der Formel

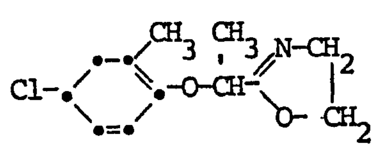

gemäss dem US Patent No. 3 877 921.

Selektive Herbizide Wirkung auf Reis im Nachauflaufverfahren

Reispflänzchen, welche 25 Tage alt sind, werden im Gewächshaus in grosse rechteckige Eternitschalen verpflanzt. Zwischen die Reihen der Reispflanzen werden dann Samen der in Reiskulturen vorkommenden Unkräuter Echinochloa crus galli, Cyperus difformis, Ammania indica und Rotala indica gesät. Die Schalen werden gut bewässert und bei einer Temperatur von ca. 25° und hoher Luftfeuchtigkeit gehalten. Nach 12 Tagen, wenn die Unkräuter aufgelaufen sind und das 2—3 Blattstadium

erreicht haben, wird die Erde in der Schale mit einer 2,5 cm hohen Schicht Wasser bedeckt. Der Wirkstoff wird dann als Emulsionskonzentrat mittels einer Pipette zwischen die Pflanzenreihen appliziert, wobei man das Emulsionskonzentrat so verdünnt und aufträgt, dass es einer Applikationsmenge im Feld von 2 und 1 kg Wirkstoff pro Hektar entspricht. Der Versuch wird nach 4 Wochen danach ausgewertet.

In diesem Versuch hat die Verbindung No. 1 die dikotylen Unkräuter Ammania indica und Rotala indica schwer sowie das Gras Cyperus difformis merklich geschädigt. Bei Echinochloa crus galli traten bloss leichte Schäden auf der Reis blieb ungeschädigt.

## Patentansprüche

1. Phenoxy-alkyl-oxazoline der Formel I

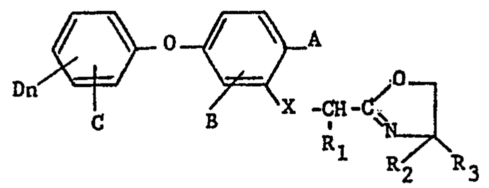

(I)

in welcher

- A Wasserstoff, ein Halogenatom, die Cyano- oder Nitrogruppe, den Amido- oder Thiamidorest —$CONH_2$ oder —$CSNH_2$,
- B Wasserstoff, ein Halogenatom oder eine $C_1$—$C_4$ Alkylgruppe,
- C Halogen, eine Cyano-, Nitro- oder Trifluormethylgruppe, den Amido oder Thiamidorest,
- D ein Halogenatom, die Cyano- oder Nitrogruppe,
- n eine Zahl 0, 1 oder 2,
- $R_1$, $R_2$ und $R_3$ unabhängig voneinander je Wasserstoff oder eine $C_1$—$C_4$ Alkylgruppe,
- X Sauerstoff, Schwefel, eine Sulfinyl- oder Sulfonylgruppe —SO— oder —$SO_2$—.

bedeuten.

2. Phenoxyalkyloxazoline gemäss Patentanspruch 1, dadurch gekennzeichnet, dass C nicht Wasserstoff bedeutet und sich in ortho-Stellung und D sich in para-Stellung zum Sauerstoffatom befinden und n 1 ist.

3. Phenoxy-alkyloxazoline gemäss Patentansprüchen 1 und 2, dadurch gekennzeichnet, dass $R_1$ Methyl und die Reste $R_2$ und $R_3$ Wasserstoff bedeuten.

4. 2 - [1 - [3′ - (2″,4″ - Dichlorphenoxy) - 6′ - chlorphenoxy] - äthyl] - oxazolin gemäss Patentanspruch 1.

5. 2 - [1 - [3′ - (2″ - Chlor - 4″ - trifluormethylphenoxy) - 6′ - chlorphenoxy] - äthyl] - oxazolin gemäss Patentanspruch 1.

6. 2 - [1 - [3′ - (2″ - Chlor - 4″ - trifluormethylphenoxy) - 6′ - nitrophenoxy] - äthyl] - oxazolin gemäss Patentanspruch 1.

7. Verfahren zur Herstellung der Phenoxy-alkyl-oxazolinen der Formel I, Anspruch 1, dadurch gekennzeichnet, dass man in einem Phenoxy-alkyl-äthylenimid der Formel II

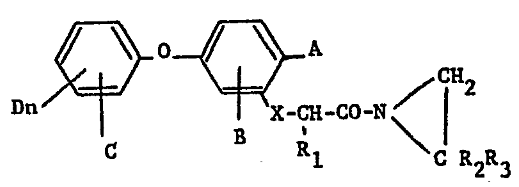

(II)

worin $R_1$, $R_2$, $R_3$, A, B, C, D und X die unter Formel I, Anspruch 1 gegebene Bedeutung haben, den Aziridinylring in einem Lösungsmittel unter Einfluss des Jodidions zum Oxazolin-Ring umlagert.

8. Verfahren zur Herstellung der Phenoxy-alkyl-oxazoline der Formel I, Anspruch 1, dadurch gekennzeichnet, dass man ein Phenoxy-alkyl-äthylamid der Formel III

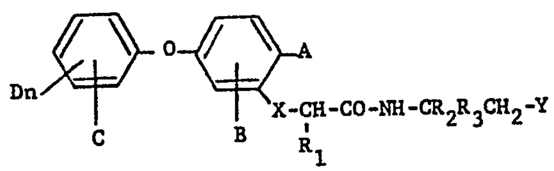

(III)

worin $R_1$, $R_2$, $R_3$, A, B, C, D und X die unter Formel I, Anspruch 1 gegebenen Bedeutung haben, während Y die OH-Gruppe, ein Halogenatom oder einen Sulfonsäurerest bedeuten kann, in einem Lösungsmittel zum Ringschluss bringt.

9. Verfahren zur Herstellung der Phenoxy-alkyl-oxazoline der Formel I, Anspruch 1, dadurch gekennzeichnet, dass man ein meta-Phenoxy-phenol der Formel IV

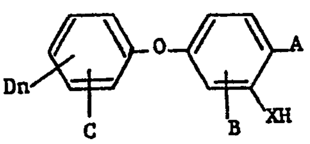

(IV)

worin A, B, C, D und X die unter Formel I, Anspruch 1 gegebene Bedeutung hat, mit einem 2 - Oxazolin - alkyl - halogenid der Formel V

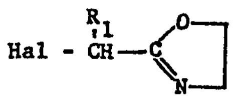

(V)

worin Hal ein Halogenatom bedeutet und $R_1$, $R_2$ und $R_3$ die unter Formel I, Anspruch 1 gegebene Bedeutung haben, in Gegenwart eines säurebindenden Mittels kondensiert.

10. Herbizides Mittel, dadurch gekennzeichnet, dass es als aktive Komponente ein Phenoxyalkyloxyzolin der Formel I, des Anspruchs 1 enthält.

11. Verfahren zur selektiven Bekämpfung von Unkräutern in monocotylen Kulturpflanzenbeständen, insbesondere in Getreide, Mais und Reis, dadurch gekennzeichnet, dass man die angesäten Kulturflächen pre-emergent, aber vorzugsweise die aufgelaufenen Kulturpflanzenbestände post-emergent mit einem herbiziden Mittel behandelt, welches als aktive Komponente ein Phenoxyalkyloxazolin der Formel I, des Anspruchs 1 enthält.

12. Verfahren gemäss Patentanspruch 11, dadurch gekennzeichnet, dass man die Kulturpflanzenbestände post-emergent mit einem herbiziden Mittel behandelt, welches als Wirkstoff 2 - [1 - [3' - (2',4'' - Dichlorphenoxy) - 6' - chlorphenoxy] - äthyl] - oxazolin enthält.

13. Verfahren gemäss Patentanspruch 11, dadurch gekennzeichnet, dass man die Kulturpflanzenbestände post-emergent mit einem herbiziden Mittel behandelt, welches als Wirkstoff 2 - [1 - [3' - (2' - Chlor - 4'' - trifluormethylphenoxy) - 6' - chlorphenoxy] - äthyl] - oxazolin enthält.

14. Verfahren gemäss Ansprüchen 11 bis 13 zur post-emergenten Bekämpfung von dicotylen Unkräutern in Sojakulturen.

**Revendications**

1. Phénoxy-alkyl-oxazolines de formule I

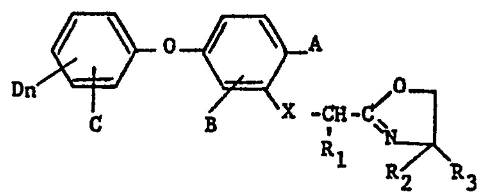

(I)

dans laquelle

A représente l'hydrogène, un atome d'halogène, le groupe cyano ou nitro, le reste amido ou thioamido —$CONH_2$ ou —$CSNH_2$,
B représente l'hydrogène, un atome d'halogène, ou un groupe alkyle en C 1—C 4,
C représente un halogène, un groupe cyano, nitro ou trifluorométhyle, le reste amido ou thiamido,
D représente un atome d'halogène, le groupe cyano ou nitro,
n est un nombre égal à 0, 1 ou 2.
$R_1$, $R_2$ et $R_3$ représentent chacun, indépendamment les uns des autres, un atome d'hydrogène ou un groupe alkyle en C 1—C 4,
X représente l'oxygène, le soufre, un groupe sulfinyle ou sulfonyle —SO— ou —$SO_2$—.

2. Phénoxy-alkyl-oxazolines selon la revendication 1, caractérisées en ce que C ne représente pas l'hydrogène et se trouve en position ortho de l'atome, d'oxygène, D est en position para de l'atome d'oxygène et n est égal à 1.

3. Phénoxy-alkyl-oxazolines selon les revendications 1 et 2, caractérisées en ce que $R_1$ représente le groupe méthyle et $R_2$ et $R_3$ représentent l'hydrogène.

4. 2 - [1 - [3' - (2'',4'' - dichlorophénoxy) - 6' - chloro - phénoxy] - éthyl]oxazoline selon la revendication 1.

5. 2 - [1 - [3' - (2'' - chloro - 4'' - trifluorométhylphénoxy) - 6' - chlorophénoxy] - éthyl] - oxazoline selon la revendication 1.

6. 2 - [1 - [3' - (2'' - chloro - 4'' - trifluorométhylphénoxy) - 6' - nitrophénoxy] - éthyl] - oxazoline selon la revendication 1.

7. Procédé de préparation des phénoxy-alkyl-oxazolines de formule I de la revendication 1, caractérisé en ce que, dans un phénoxy - alkyl - éthylène - imide de formule II

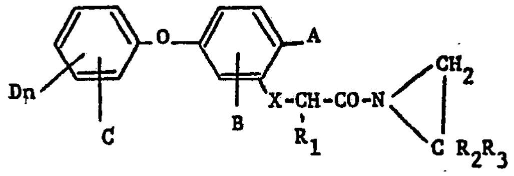

(II)

dans laquelle $R_1$, $R_2$, $R_3$, A, B, C, D et X ont les significations indiquées en référence à la formule I de la revendication 1, on transpose le cycle aziridinyle en cycle oxazoline dans un solvant sous l'influence de l'ion iodure.

8. Procédé de préparation des phénoxy-alkyl-oxazolines de formule I selon la revendication 1, caractérisé en ce que l'on cyclise un phénoxy - alkyl - éthylamide de formule III

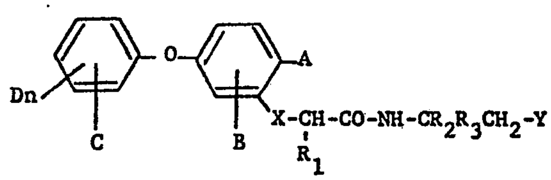

(III)

dans laquelle $R_1$, $R_2$, $R_3$, A, B, C, D et X ont les significations indiquées en référence à la formule I de la revendication 1, et Y représente la groupe OH, un atome d'halogène ou un reste d'acide sulfonique, dans un solvant.

9. Procédé de préparation des phénoxy-alkyl - oxazolines de formule I de la revendication 1, caractérisé en ce que l'on condense en présence d'un agent fixant les acides un métaphénoxy-phénol de formule IV

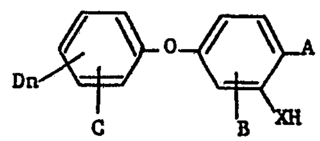

(IV)

dans laquelle A, B, C, D et X ont les significations indiquées en référence à la formule I de la revendication 1, avec un halogénure de 2 - oxazoline - alkyle de formule V

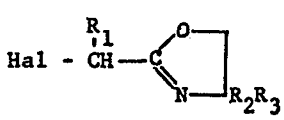

(V)

dans laquelle Hal représente un atome d'halogène et $R_1$, $R_2$ et $R_3$ ont les significations indiquées en référence à la formule I de la revendication 1.

10. Produit herbicide caractérisé en ce qu'il contient en tant que composant actif une phénoxyalkyloxazoline de formule I de la revendication 1.

11. Procédé pour combattre sélectivement les mauvaises herbes dans les cultures de végétaux monocotylédones, en particulier de céréales, de maïs et de riz, caractérisé en ce que l'on traite les surfaces cultivées ensemencées avant sortie de terre mais de préférence les cultures de végétaux développés, après sortie de terre, par un produit herbicide contenant en tant que composant actif une phénoxyalkyloxazoline de formule I de la revendication 1.

12. Precédé selon la revendication 11, caractérisé en ce que l'on traite les cultures après sortie de terre par un produit herbicide contenant en tant que substance active la 2 - [1 - [3' - (2',4'' - dichlorophénoxy) - 6' - chlorophénoxy] - éthyl] - oxazoline.

13. Procédé selon la revendication 11, caractérisé en ce que l'on traite les cultures après sortie de terre par un produit herbicide contenant en tant que substance active la 2 - [1 - [3' - (2' - chloro - 4'' - trifluorométhylphénoxy) - 6' - chlorophénoxy] - éthyl] - oxazoline.

14. Procédé selon les revendications 11 à 13 pour la lutte après sortie de terre contre les mauvaises herbes dicotylédones dans les cultures de soja.

**Claims**

1. A phenoxyalkyloxazoline of the formula I

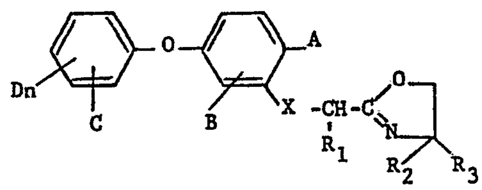

(I)

wherein

A represents hydrogen, a halogen atom, the cyano or nitro group, the amido or thiamido radical $—CONH_2$ or $—CSNH_2$,
B represents hydrogen, a halogen atom or a $C_1—C_4$ alkyl group,
C represents halogen, a cyano, nitro or trifluoromethyl group, the amido or thiamido radical,
D represents a halogen atom, the cyano or nitro group,
n is 0, 1 or 2,
$R_1$, $R_2$ and $R_3$, each independently of the other, represent hydrogen or a $C_1—C_4$ alkyl group, and
X represents oxygen, sulfur, a sulfinyl or sulfonyl group $—SO—$ or $—SO_2—$.

2. A phenoxyalkyloxazoline according to claim 1, wherein C does not represent hydrogen and is the in the ortho- position to the oxygen atom and D is in the para-position to the oxygen atom, and n is 1.

3. A phenoxyalkyloxazoline according to claims 1 and 2, wherein $R_1$ represents methyl and $R_2$ and $R_3$ represent hydrogen.

4. 2 - [1 - [3' - (2'',4'' - Dichlorophenoxy) - 6 - chlorophenoxy] - ethyl] - oxazoline according to claim 1.

5. 2 - [1[3' - (2'' - chloro - 4'' - Trifluoromethylphenoxy) - 6' - chloro - phenoxy] - ethyl] - oxazoline according to claim 1.

6. 2 - [1 - [3' - (2'' - Chloro - 4'' - trifluoromethylphenoxy) - 6' - nitrophenoxy] - ethyl] - oxazoline according to claim 1.

7. Process for the production of a phenoxyalkyloxazoline of the formula I of claim 1, which process comprises rearranging the aziridinyl ring in a phenoxyalkylethyleneimide of the formula II

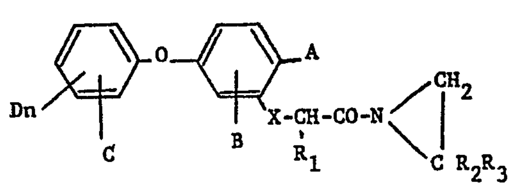

(II)

wherein $R_1$, $R_2$ $R_3$, A, B, C, D and X are as defined for formula I in claim 1, in a solvent, under the influence of the iodide ion, to form the oxazoline ring.

8. A process for the production of a phenoxyalkyloxazoline of the formula I of claim 1, which process comprises cyclising a phenoxyalkylethylamide of the formula III

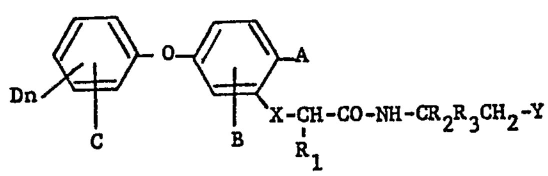

(III)

wherein $R_1$, $R_2$, $R_3$, A, B, C, D and X are as defined for formula I in claim 1, whilst Y can represent the OH group, a halogen atom or a sulfonic acid radical, in a solvent.

9. A process for the production of a phenoxyalkyloxazoline of the formula I in claim 1, which process comprises condensing a meta-phenoxyphenol of the formula IV

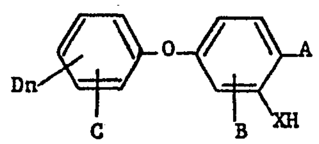

(IV)

wherein A, B, C, D and X are as defined for formula I in claim 1, with a 2-oxazoline alkyl halide of the formula V

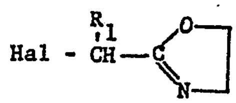

(V)

wherein Hal represents a halogen atom and $R_1$, $R_2$, $R_3$ are as defined for formula I in claim 1, in the presence of an acid acceptor.

10. A herbicidal composition which contains, as active component, a phenoxyalkyloxazoline of the formula I of claim 1.

11. A pre-emergence, preferably post-emergence, method of selectively controlling weeds in crops of monocotyledonous cultivated plants, especially in cereals, maize and rice, which comprises treating the areas under cultivation with a herbicidal composition which contains, as active component a phenoxyalkyloxazoline of the formula I of claim 1.

12. A method according to claim 11, which comprises treating the areas under cultivation post-emergence with a herbicidal composition which contains, as active component, 2 - [1 - [3′ - (2′,4″ - dichlorophenoxy) - 6′ - chlorophenoxy] - ethyl] - oxazoline.

13. A method according to claim 11, which comprises treating the areas under cultivation post-emergence with a herbicidal composition which contains, as active component 2 - [1 - [3′ - (2′ - chloro - 4″ - trifluoromethylphenoxy) - 6′ - chlorophenoxy] - ethyl] - oxazoline.

14. A method according to claims 11 to 13 for the post-emergence control of dicotyledonous weeds in soya crops.